# EUROPEAN PATENT APPLICATION

(11) **EP 3 485 893 A1**
(43) Date of publication of application: **22.05.2019**
(21) Application number: 17830129.7
(22) Date of filing: 17.07.2017
(51) Int. Cl.: A61K 36/36, A61P 29/00

(54) **USE OF A CLOVE OIL FRACTION**

(30) Priority: 18.07.2016 BR 102016016555
(71) Applicant: Castilho, André Moreira, CEP-30310-580 Belo Horizonte, MB (BR)
(72) Inventor: Pianowski, Luiz Francisco, CEP: 12917-025 Bragança Paulista - SP (BR)
(74) Representative: KATZAROV S.A.
(86) International application number: PCT/BR2017/050194
(87) International publication number: WO 2018/014101

(57) **Abstract**

The present invention relates to the use of a clove oil fraction in the preparation of a product useful in the treatment of inflammatory conditions of the animal body, such as analgesic and anti-inflammatory, particularly a fraction remaining after removal of eugenol.

## Description

The present invention relates to the use of a clove oil fraction in the preparation of a product useful in the treatment of inflammatory conditions of the animal body, such as analgesic and anti-inflammatory, particularly a fraction remaining after removal of eugenol.

### Background of the invention

The essential oil of clove (*Syzygium aromaticum*, *Cariophylus arimathicus* L, *Eugenia aromatica* (L.) Baill., *Eugenia caryophyllata* Thunb., *Eugenia caryophyllus* (Spreng.)), also commonly called clove, is known and used in folk medicine. Its main constituent is eugenol, in typical contents of 70-90%.

Eugenol is an aromatic compound, named as 4-allyl-2-methoxyphenol, and anti-inflammatory, healing, antiseptic and analgesic properties are assigned to it. However, clove oil is commonly cited as a synonym for eugenol for it is present in high content, thus certain amounts of clove oil are not derived from eugenol.

Eugenol is widely used in dentistry, as an anesthetic in the dental area, for the relief of toothaches. In this sense, eugenol is extracted from clove oil and the rest is discarded.

However, other components of clove oil also have interesting characteristics, such as transcariophyllene (or beta-caryophyllene) and alpha-humulene, at reported amounts of 5-12% and 1-1.5% of the total oil, respectively.

Such transcariophyllene and alpha-humulene compounds are known to be effective in treating inflammatory conditions of the animal body, particularly inflammatory pain or inflammatory edema.

### Detailed description of the invention

In view of the above facts, this invention relates to the use of a clove oil fraction in the preparation of a product useful in the treatment of inflammatory conditions of the animal body, said fraction being a fraction remaining after removal of eugenol.

In particular, the mention of the animal body refers to the human body.

As used herein, "a fraction remaining after removal of eugenol" refers to any fraction, from that which has minimally removed eugenol from clove oil, or fractions obtained therefrom, to remove additional components, except transcaryophyllene and or alpha-humulene.

Within a particular embodiment of the invention, without excluding any other, the use of said product useful in the treatment of inflammatory conditions of the animal body allows the preparation of salts and derivatives of alpha-humulene and transcariophyllene.

Within a particular embodiment of the invention, without excluding any other, said product useful in the treatment of inflammatory conditions of the animal body refers to conditions that cause inflammation, inflammatory pain and inflammatory edema.

Within a particular embodiment of the invention, without excluding any other, said product useful in the treatment of inflammatory conditions of the animal body is an additive to a medicament, an adjuvant to a medicament or a medicament itself.

Within embodiments of the invention, without excluding any other, said product useful in the treatment of inflammatory conditions of the body prepared from the use of the clove oil fraction is suitable for enteral or parenteral administration, including topical, transdermal, subcutaneous, intraperitoneal, intravenous, by infiltration, by inhalation, trans-mucosal, intramuscular, intrapulmonary, vaginal, rectal, intraocular and sublingual.

Within embodiments of the invention, without excluding any other, said product useful in the treatment of inflammatory conditions of the body prepared from the use of the clove oil fraction may be in the form of solid, liquid, semisolids or pastes, pills, tablets, hard or soft capsules, lozenges, powders, granules, suspensions, dispersions, emulsions, and any other form known to the person skilled in the art.

Within some embodiments of the invention, said product may be of immediate or extended release, and may make use of micro or nanoparticles.

Within embodiments of the invention, without excluding any other, some of the inflammatory conditions of said product prepared from the use of the clove oil fraction are one or more of rheumatoid arthritis, osteoarthritis, systemic lupus erythematosus, ulcerative colitis, psoriasis, atopic eczema, atherosclerosis, non-degenerative diseases e.g. depression, cellulitis and allergy.

The product prepared from the use of the clove oil fraction according to the invention typically contains excipients known to a person skilled in the art, for example those mentioned in the publication "Remington - The Science and Practice of Pharmacy" by Lippincott Williams & Wilkins, 2006.

Within embodiments of the invention, without excluding any other, said product useful in the treatment of inflammatory conditions of the body prepared from the use of the clove oil fraction, in use for oral ingestion, contains from 1 mg to 1000 mg of the transcariophyllene and alpha-humulene mixture as contained in the clove oil fraction upon removal of eugenol, particularly between 10 and 100 mg, more particularly from 30 to 100 mg.

For topical use, without excluding any other alternative, the said product contains 0.01 to 100% of the clove oil fraction.

Within a particular embodiment of the invention, said product useful in the treatment of inflammatory conditions of the body prepared from the use of the clove oil fraction may contain other assets than those contained in said fraction, e.g. one or more of an antibiotic, muscle relaxant, hormone, antihyperlipidemic, antiallergic, vitamins, calcium sources, antidepressant, healing, etc.

One skilled in the art may, from the information provided herein, carry out the invention of equivalent forms not expressly described, but with the same or substantially the same function, and the same or substantially the same results, while still being within the scope of protection of the appended claims.

## Claims

1. Use of a clove oil fraction **characterized by** being in the preparation of useful product in the treatment of inflammatory conditions of the animal body, said fraction being a remaining fraction after removal of eugenol.

2. Use according to claim 1 **characterized by** said product useful in the treatment of inflammatory conditions of the animal body referring to conditions that cause inflammation, inflammatory pain or inflammatory edema.

3. Use according to claim 1 **characterized by** said product useful in the treatment of inflammatory conditions of the animal body being an additive to a medicament, an adjuvant to a medicament or a medicament itself.

4. Use according to claim 1 **characterized by** said product useful in the treatment of inflammatory conditions of the animal body being suitable for enteral or parenteral administration including topical, transdermal, subcutaneous, intraperitoneal, intravenous, by infiltration, by inhalation, trans-mucosal, intramuscular, intrapulmonary, vaginal, rectal, intraocular and sublingual.

5. Use according to claim 1 **characterized by** said product useful in the treatment of inflammatory conditions of the animal body being presented in the form of solid, liquid, semisolid or pastes, pills, tablets, capsules, lozenges, powders, granules, suspensions, dispersions, emulsions.

6. Use according to claim 1 **characterized by** said product useful in the treatment of inflammatory conditions of the animal body referring to the inflammatory condition of rheumatoid arthritis, osteoarthritis, systemic erythematosus lupus, ulcerative colitis, psoriasis, atopic eczema, atherosclerosis, non-degenerative diseases e.g. depression, cellulitis or allergy.

7. Use according to claim 1 **characterized by** said product useful in the treatment of inflammatory conditions of the animal body containing from 1 mg to 1000 mg of the transcariophyllene and alpha-humulene mixture contained in the clove oil fraction after removal of eugenol, particularly from 10 to 100mg, more particularly from 30 to 100 mg.

8. Use according to claim 1 **characterized by** said product useful in the treatment of inflammatory conditions of the animal body containing one or more assets other than those contained in said fraction.

9. Use according to claim 9 **characterized by** said product useful in the treatment of inflammatory conditions of the animal body containing one or more active other than those contained in said fraction, such as antibiotic, muscle relaxant, hormone, antihyperlipidemic, antiallergics, vitamins, calcium sources, antidepressant, healing.
